# EUROPÄISCHE PATENTSCHRIFT

(11) **EP 3 020 432 B1**
(45) Veröffentlichungstag und Bekanntmachung des Hinweises auf die Patenterteilung: **28.07.2021**
(21) Anmeldenummer: 15187719.8
(22) Anmeldetag: 30.09.2015
(51) Int. Cl.: A61M 5/31

(54) **SPRITZE MIT FINGERFLANSCH SOWIE FINGERFLANSCH**
SYRINGE WITH FINGER FLANGE AND FINGER FLANGE
SERINGUE DOTEE DE BRIDE POUR LES DOIGTS ET BRIDE POUR LES DOIGTS

(30) Priorität: 11.11.2014 DE 102014116396
(43) Veröffentlichungstag der Anmeldung: 18.05.2016
(73) Patentinhaber: SCHOTT Schweiz AG, 9001 St. Gallen (CH)
(72) Erfinder: KOMANN, Christian, 9000 St. Gallen (CH)
(74) Vertreter: Blumbach · Zinngrebe Patentanwälte PartG mbB

(56) Entgegenhaltungen:
- WO-A1-2011/122351
- WO-A1-2011/133097
- WO-A1-2013/009387
- DE-A1-102004 017 004
- DE-A1-102006 005 784
- DE-B4- 4 323 124
- None

## Beschreibung

Die Erfindung betrifft eine Spritze mit einem Fingerflansch und einer Fingerauflage. Ferner betrifft die Erfindung einen derartigen Fingerflansch.

Spritzen mit einem länglichen, zylinderförmigen Spritzenkörper, in dem eine in Richtung der Längsachse bewegliche Kolbenstange angeordnet ist, sind allgemein bekannt. Derartige Spritzen sind üblicherweise aus Kunststoff oder Glas hergestellt. Sie weisen im allgemeinen an dem distalen Ende des Spritzenkörpers einen konusartigen Spritzenansatz auf, der mit einem Nadelträger oder einer Vorrichtung zur Aufnahme einer Nadel verbunden werden kann. Ferner weist der Spritzenkörper im allgemeinen ein proximales offenes Ende auf, in das die Kolbenstange eingeführt werden kann.

Das in dem Spritzenkörper ausgebildete Innenvolumen kann mit einer Substanz gefüllt werden. Durch eine Hubbewegung der Kolbenstange kann die Substanz an dem distalen Ende des Spritzenkörpers durch einen Durchlass aus diesem ausgetrieben werden. Zur Erleichterung der Handhabung während der Hubbewegung ist häufig ein sogenannter Fingerflansch vorgesehen, welcher eine Auflage für Finger aufweist.

In vielen Fällen ist der Fingerflansch einstückig zusammen mit dem Spritzenkörper ausgebildet. Dieses ist beispielsweise bei einem Spritzenkörper aus Kunststoff relativ einfach möglich. Werden dagegen Spritzenkörper aus Glas verwendet, so ist das Ausbilden eines geeigneten Fingerflansches aus dem Glaskörper engen konstruktiven Grenzen gesetzt. Dieses gilt umso mehr, wenn der Spritzenkörper beispielsweise aus einem Stück Glasrohr gefertigt wird. Um eine hinreichende Festigkeit des Fingerflansches zu gewährleisten, sind größere Umformungen des Glasrohrs oder auch eine Fertigung mit Übergröße erforderlich.

Um dieses zu vermeiden, sind verschiedene Fingerflansche zur nachtäglichen Montage an einer Spritze entwickelt worden.

So ist beispielsweise der Patentschrift DE 43 23 124 B4 eine Spritze mit einem Fingerflansch zu entnehmen, welcher auf den Spritzenkörper axial aufgeschoben werden kann. Der Fingerflansch ist dabei derart ausgebildet, dass er eine Öffnung aufweist, die so bemessen ist, dass sie beim Aufschieben aufgedrückt wird und anschließend in einem dafür vorgesehenen Bereich einrastet. Als ungünstig kann bei diesem Verfahren der hohe Aufwand angesehen werden, der an die Genauigkeit der Außengeometrie des Spritzenkörpers gestellt wird, so dass ein Aufschieben des Fingerflansches überhaupt erst ermöglicht wird. Durch das Aufschieben erfolgt eine Aufweitung der Öffnung des Fingerflansches.

Eine andere Form eines nachträglich an einer Spritze montierbaren Fingerflansches offenbart die DE 10 2004 036 051 A1. Es wird ein Fingerflansch vorgeschlagen, welcher an dem distalen Ende des Spritzenkörper montiert werden kann durch ein seitliches Aufschieben in einem dafür vorgesehenen Bereich des Spritzenkörpers. Der hier vorgestellte Fingerflansch ist zwar einfach zu montieren. Allerdings kann er auch einfach demontiert werden bzw. er ist nicht gegen ein unbeabsichtigtes Lösen gesichert. Zudem ist eine Verdrehung zum Spritzenkörper möglich, so dass eine Handhabung des Fingerflansches erschwert werden kann.

Ferner offenbart das Dokument DE 10 2006 005 784 eine Spritze mit einem zylinderförmigen Spritzenkörper, bei welchem ein mehrteiliges Bauteil vorgesehen ist, welches als Fingerflansch ausgebildet ist. Der Fingerflansch kann mit dem proximalen Ende des Spritzenkörpers verbunden werden und ist dazu ausgebildet, nicht ohne Beeinträchtigung des Spritzenkörpers abgenommen werden zu können. Ein Nachteil dieser Ausführungsform ist der mehrteilige Aufbau, der zu einem erhöhten Aufwand bzgl. Lagerhaltung und Logistik sowie einer komplizierten Montage führt.

Das Dokument DE 10 2004 017 004 A1 offenbart eine mehrteilige Vorrichtung zum Halten einer angiographischen Spritze.

Das Dokument WO 2013/009387 A1 offenbart einen Fingerflansch für eine Spritze mit einem zumindest zweiteiligen Aufbau des Fingerflansches. Die beiden Teile werden von gegenüberliegenden Seiten radial auf die Spritze aufgeschoben.

Das Dokument WO 2011/133097 A1 offenbart ein Bauteil zum Halten einer Kolbenstange in einer bestimmten Position innerhalb einer Spritze. Dieses Bauteil ist als mehrteilige Gewindemutter ausgebildet. Die Gewindemutter kann einfach demontiert werden, um die Fixierung zu lösen.

Das Dokument WO 2011/122351 A1 offenbart eine Spritze mit einem Fingerflansch, der mittels einer lösbaren Rastverbindung befestigt wird.

Hieraus ergibt sich die Aufgabe der vorliegenden Erfindung, die darin zu sehen ist, eine Spritze mit einem Fingerflansch zur Verfügung zu stellen, wobei der Fingerflansch nachträglich an die Spritze montiert werden kann. Ferner soll ein entsprechender Fingerflansch zur Verfügung gestellt werden.

Dabei soll der Fingerflansch fest mit der Spritze verbunden werden können, ohne dass er wieder demontiert werden kann. Ferner soll er auch im montierten Zustand verdrehsicher an der Spritze halten. Eine Montage des Fingerflansches soll auch bei geringen Form- und Lageabweichungen der Spritze, insbesondere im Bereich des proximalen Endes, möglich sein. Die Fertigung des Fingerflansches soll kostengünstig und in großen Stückzahlen möglich sein. Daher soll der Fingerflansch möglichst einstückig ausgebildet sein, bevorzugt aus einem einzigen Material, und/oder mittels eines einfachen, auf große Stückzahlen ausgerichteten Fertigungsverfahrens herstellbar sein.

Überraschend einfach wird diese Aufgabe durch eine Spritze sowie einen Fingerflansch nach einem der unabhängigen Ansprüche gelöst. Bevorzugte Ausführungsformen und Weiterbildungen der Erfindung sind den jeweiligen Unteransprüchen zu entnehmen.

Die Erfindung betrifft demnach eine Spritze, umfassend einen zylinderförmigen Spritzenkörper mit einem distalen Ende und einem proximalen Ende sowie einen Fingerflansch mit zumindest einer Auflagefläche für einen Finger, wobei
- der Spritzenkörper eine zylinderförmige Kammer umfasst zur Aufnahme einer flüssigen Substanz,
- das proximale Ende des Spritzenkörpers eine Öffnung aufweist, durch die eine Kolbenstange geführt werden kann,
- das proximale Ende des Spritzenkörpers ferner zumindest einen radial nach außen vorspringenden, über die Mantelfläche des Spritzenkörpers vorstehenden Vorsprung umfasst,
- der Fingerflansch einstückig ausgebildet ist,
- der Fingerflansch zumindest ein erstes und ein zweites Fingerflanschelement umfasst, welche beweglich miteinander verbunden sind und von einer geöffneten Position in eine geschlossene Position bewegt werden können,
- der Fingerflansch in geschlossener Position zumindest teilweise mit dem zumindest einen Vorsprung kraftschlüssig verbunden ist und den Vorsprung zumindest teilweise derart umschließt, dass in geschlossener Position ein axiales Verschieben des Fingerflansches relativ zu dem Spritzenkörper verhindert wird, und wobei
- der Fingerflansch eine Ausnehmung zur Durchführung der Kolbenstange aufweist.

Das distale Ende des Spritzenkörpers ist dabei ausgebildet zur Verbindung mit einer Nadel oder einer

Nadelaufnahmevorrichtung. Der Spritzenkörper umfasst ferner eine Kammer zur Aufnahme einer flüssigen Substanz oder eines Fluids. Die Spritze kann sowohl ungefüllt, aber auch bereits mit der Substanz gefüllt sein. Im gefüllten Zustand ist im allgemeinen ein Stopfen vorgesehen, welcher die Kammer fluiddicht verschließt, um ein ungewolltes Austreten der Substanz zu vermeiden. Das distale Ende der Spritze kann einen Durchlass zum Austreiben der Substanz aufweisen. Das proximale Ende des Spritzenkörpers weist ferner eine Öffnung auf, durch die eine Kolbenstange in die Kammer eingeführt werden kann.

Im Bereich des proximalen Endes kann der Spritzenkörper mit zumindest einem nach außen vorstehenden Vorsprung, der somit über die äußere Mantelfläche des Spritzenkörpers hervorsteht, ausgebildet sein. Dies kann beispielsweise ein vorstehender ringförmiger oder ringförmig umlaufender Vorsprung bzw. auch ein ringförmig umlaufender Wulst sein. Der Spritzenkörper kann auch an seinem proximalen Ende eine Nut umfassen.

Der Fingerflansch kann im Sinne der Erfindung mit dem proximalen Ende des Spritzenkörpers kraftschlüssig und/oder formschlüssig verbunden sein. Bevorzugt ist er in der geschlossenen Position dabei gleichzeitig gegen ein ungewolltes Lösen gesichert. Dabei kann der Fingerflansch zumindest ein erstes und ein zweites Fingerflanschelement umfassen, welche beweglich zueinander angeordnet sind und von einer geöffneten Position in eine geschlossene Position bewegt werden können. Bevorzugt dient die geöffnete Position einer einfachen Montage des Fingerflansches an dem Spritzenkörper, wobei durch eine Schließbewegung sodann die formschlüssige Verbindung geschaffen werden kann.

Durch die kraft- und/oder formschlüssige Verbindung, welche in geschlossener Position des Fingerflansches zwischen diesem und dem Spritzenkörper geschaffen wird, wird eine axiale Bewegung des Fingerflansches relativ zu dem Spritzenkörper, also eine Relativbewegung parallel zur Mittenlinie des Spritzenkörpers, verhindert. Dabei kann zumindest ein Teil des Fingerflansches und/oder des Fingerflanschelements den Spritzenkörper an seinem proximalen Ende zumindest teilweise umgeben oder auch vollständig umschließen.

Der Fingerflansch weist weiterhin zumindest eine bevorzugt durchgehende Ausnehmung auf, welche in montierter Position vorteilhaft deckungsgleich zur Öffnung des Spritzenkörpers angeordnet ist, so dass bei einem an dem Spritzenkörper montierten Fingerflansch die Durchführung der Kolbenstange ermöglicht wird. Diese Ausnehmung kann auch eine durchgehende Öffnung sein.

Durch die Öffnung im proximalen Ende des Spritzenkörpers und durch die durchgehende Ausnehmung oder Öffnung im Fingerflansch kann eine Kolbenstange in die Kammer eingeführt werden. Diese kann, beispielweise bei einer mit einer Substanz gefüllten Spritze, dazu verwendet werden, den Stopfen zu bewegen. Hierzu kann die Kolbenstange beispielsweise in den Stopfen in ein dafür vorgesehenes Gewinde eingedreht und mit diesem somit fest verbunden werden.

In einer anderen Ausführungsform ist die Spritze nicht gefüllt. Die Kolbenstange kann dann mit einem die Kammer fluiddicht abschließenden Endstück ausgebildet sein, so dass durch eine axiale Hubbewegung in Richtung des proximalen Endes eine Substanz durch die Öffnung in dem distalen Ende in die Kammer des Spritzenkörpers eingezogen werden kann, welche dann später durch eine umgekehrte Hubbewegung der Kolbenstange in Richtung des distalen Endes wieder ausgetrieben werden kann. Es kann aber auch eine Kolbenstange mit einem daran befestigten Stopfen in die Kammer eingeführt werden.

Die Montagereihenfolge von Fingerflansch und Kolbenstange kann unterschiedlich sein. So können zunächst der Fingerflansch und dann die Kolbenstange montiert werden, aber auch eine umgekehrte Montagereihenfolge ist möglich.

In vorteilhafter Weise ist die Ausnehmung des Fingerflansches dabei elastisch oder teilelastisch ausgeführt, so dass die Kolbenstange zwar eingeführt, aber nicht mehr nachträglich ausgezogen werden kann. Dies kann beispielsweise durch eine sich bei einem Einschieben der Kolbenstange nur in eine Richtung aufweitende elastische Ausnehmung ermöglicht werden, welche nach dem Einschieben zurückschnappt und ein Herausziehen verhindert. Diese Auszugsicherung der Kolbenstange wird auch als "Backstop"-Funktion bezeichnet.

Besonders günstig ist diese Ausgestaltung bei einer Montagereihenfolge, bei der die Kolbenstange nach dem Fingerflansch montiert wird. Durch die Backstop-Funktion des Fingerflansches kann auf einfache Weise sichergestellt werden, dass die Kolbenstange unbeabsichtigt wieder entfernt und die Spritze unbrauchbar gemacht wird.

In einer anderen Ausführungsform ist die Ausnehmung des Fingerflansches derart ausgeführt, dass ein Herausziehen des Stopfens verhindert wird. Diese Backstop-Funktion dient demnach als Auszugsicherung des Stopfens. Die Kolbenstange kann bei dieser Ausführungsform kleiner oder dünner ausgebildet sein.

Ferner umfasst der Fingerflansch zumindest eine Auflagefläche für einen Finger, welche zur Auflage eines Fingers radial nach außen hervorsteht. Bevorzugt sind zumindest zwei, sich gegenüberliegende Fingerauflagen vorgesehen, so dass eine besonders sichere und stabile Auflagemöglichkeit und damit eine sichere Handhabbarkeit geschaffen werden kann.

Ein Fingerflansch im Sinne der Erfindung umfasst zumindest ein erstes und ein zweites Fingerflanschelement, welche beweglich, bevorzugt drehbeweglich, miteinander verbunden sind.

Erfindungsgemäß ist der Fingerflansch einstückig ausgebildet. Hierunter ist insbesondere zu verstehen, dass der Fingerflansch als ein einziges Bauteil zur Montage an dem Spritzenkörper bereitgestellt werden kann. Hierdurch ist eine kostengünstige Fertigung in großer Stückzahl möglich. Zudem können Kosten für Transport und Logistik minimiert werden. Die Fingerflanschelemente des Fingerflansches sind dabei derart miteinander verbunden, dass ein Trennen nur unter Überwindung eines Widerstands möglich ist, um ein ungewolltes oder unbeabsichtigtes Trennen auszuschließen. Besonders bevorzugt sind die Fingerflanschelemente nicht zerstörungsfrei voneinander trennbar. Hierdurch kann sichergestellt werden, dass ein bereits montierter Fingerflansch nicht wieder demontiert und anschließend erneut montiert werden kann.

Diese zumindest zwei Fingerflanschelemente des einstückigen Fingerflansches können dabei mittels eines Gelenks, bevorzugt mittels eines Drehgelenks, formschlüssig miteinander verbunden sein. Auf diese Weise können die Fingerflanschelemente beweglich, bevorzugt drehbeweglich, miteinander verbunden sein.

In einer besonders bevorzugten Ausführungsform können die Fingerflanschelemente des einstückigen Fingerflansches auch stoffschlüssig miteinander verbunden sein, wobei ebenfalls eine Beweglichkeit mittels eines Gelenks hergestellt sein kann.

Bevorzugt sind die Fingerflanschelemente drehbeweglich miteinander verbunden, so dass eine Drehbewegung des zumindest einen Fingerflanschelements relativ zu dem zumindest einen weiteren Fingerflanschelement ermöglicht wird.

Die Drehachse der Fingerflanschelemente kann dabei entlang bzw. parallel zu einer gemeinsamen geraden Kante verlaufen. Diese Drehachse kann - im montierten Zustand - sowohl parallel als auch senkrecht zu der Mittenlinie des Spritzenkörpers stehen. Durch eine Drehbewegung der Fingerflanschelemente relativ zueinander kann der Fingerflansch, insbesondere nach der Montage an dem Spritzenkörper, von einer offenen in eine geschlossene Position gebracht werden. Hierdurch können die Fingerflanschelemente zumindest eine gemeinsame Kontaktfläche ausbilden.

Auf diese Weise kann der Fingerflansch beispielsweise im geöffneten Zustand zur Anbringung an den Spritzenkörper zunächst mit dem ersten Fingerflanschelement durch Aufstecken, Aufschieben oder Klemmen mit dem Spritzenkörper verbunden werden, und sodann kann durch eine einfache Drehbewegung des zweiten Fingerflanschelements der Fingerflansch in eine geschlossene Position gebracht werden, so dass eine formschlüssige Verbindung mit zumindest dem den Vorsprung umfassenden Bereich des proximalen Endes des Spritzenkörpers hergestellt werden kann.

Zur Erzeugung einer beweglichen, bevorzugt drehbeweglichen, Verbindung zwischen den Fingerflanschelementen kann ein Gelenk vorgesehen sein.

Bei einer Ausführungsform des Fingerflansches mit stoffschlüssig verbundenen Fingerflanschelementen kann das Gelenk ein biegbarer Falz oder eine dünnwandige Verbindung sein, welche eine Drehbewegung ermöglicht. Ganz besonders bevorzugt handelt es sich dabei um ein Folien- oder Filmgelenk bzw. ein Filmscharnier. Hierbei kann das Gelenk aus dem gleichen Material hergestellt sein wie der Fingerflansch, so dass eine besonders kostengünstige Produktion im Spritzgussverfahren in großen Stückzahlen ermöglicht wird.

Der einstückige Fingerflansch im Sinne der Erfindung kann aber auch aus zumindest zwei Fingerflanschelementen, welche derart miteinander verbunden werden, so dass sie nur unter Überwindung eines Widerstands getrennt werden können, gebildet werden. Dabei können die Fingerflanschelemente formschlüssig mittels eines Gelenks miteinander verbunden werden.

Die Fingerflanschelemente können aus einem ersten Material, welches fest ist oder keine oder nur eine geringe Elastizität aufweist, hergestellt sein, wobei dann für das Gelenk ein zweites Material, welches die für die Drehbewegung erforderliche Elastizität bereitstellt, vorgesehen sein kann. Ein derartiger Fingerflansch kann beispielsweise durch ein Mehrfachkomponenten-Spritzgießen sehr kostengünstig und in großen Stückzahlen erzeugt werden.

Auch eine Erzeugung des Fingerflansches nach dem In-Mould-Verfahren, bei dem zwei oder mehr Bauteile bzw. Fingerflanschelemente gemeinsam erzeugt werden, wird als im Sinne der Erfindung angesehen. Bei einem derartig erzeugten Fingerflansch werden daher auch zwei formschlüssig miteinander verbundene Fingerflanschelemente als einstückig im Sinne der Erfindung betrachtet. Auch bei diesem Verfahren ist eine kostengünstige Fertigung mit sehr hohen Stückzahlen möglich.

Auf diese Weise kann der Fingerflansch auch in dem Kontaktbereich mit dem Spritzenkörper oder in dem Bereich mit der Ausnehmung ein Material mit einer größeren Elastizität umfassen als in den übrigen Bereichen. So können die Fingerflanschelemente beispielsweise aus PP hergestellt sein und einen Elastomer im Bereich der Ausnehmung und/oder im Bereich der drehbeweglichen Verbindung bzw. des Gelenks umfassen. Hierdurch können geometrische Abweichungen des Spritzenkörpers kompensiert werden.

Auf diese Weise kann der Fingerflansch einstückig hergestellt werden, wobei im montierten Zustand eine sehr feste und stabile Verbindung mit dem Spritzenkörper hergestellt werden kann. Gleichzeitig kann aber auch die erforderliche Beweglichkeit der Fingerflanschelemente zueinander gewährleistet werden.

Vorteilhaft ist der Fingerflansch, zumindest in dem Bereich des Gelenks, aus einem teilweise elastischen oder teilelastischen Material hergestellt, um die Beweglichkeit zu gewährleisten. Hierzu können die bekannten, gängigen Materialien verwendet werden, umfassend PP, PE, POM, PBT, PA, PES, TPE, aber auch beispielsweise glasfaserverstärkte Kunststoffe. Auf diese Weise kann der Fingerflansch besonders einfach mittels gängiger Verfahren wie dem Spritzguss hergestellt werden.

In einer bevorzugten Ausführungsform ist dabei das zumindest erste Fingerflanschelement mit einer Ausnehmung, bevorzugt mit einer U-förmigen Ausnehmung, ausgebildet, um zumindest teilweise mit dem den Vorsprung umfassenden Bereich des proximalen Endes des Spritzenkörpers formschlüssig verbunden zu werden. Vorteilhaft ist daher der Kontaktbereich des Fingerflanschelements, insbesondere in dem Bereich der Ausnehmung, mit einer Innenkontur ausgebildet, welche zumindest teilweise passgenau gegengleich zu der Außenkontur des proximalen Endes des Spritzenkörpers ist.

Bevorzugt ist sie passgenau gegengleich zu dem Vorsprung oder der Nut ausgebildet. In anderen Worten, der Kontaktbereich zumindest eines Fingerflanschelementes ist der Außenkontur des proximalen Endes des Spritzenkörpers, insbesondere dem Vorsprung, derart angepasst, dass der Fingerflansch in geschlossener Position eine kraft- und/oder formschlüssige Verbindung zwischen Fingerflansch und Spritzenkörper ermöglicht.

Dabei kann das zumindest eine Fingerflanschelement die Umfangsfläche des Spritzenkörpers in einem vorbestimmten Bereich umfassen. Dies ist etwa dann vorteilhaft, wenn der Vorsprung auf dem Spritzenkörper zumindest teilweise ringförmig umlaufend ist. In einer günstigen Ausführungsform, welche besonders montagefreundlich ist, umschließt zumindest das erste Fingerflanschelement die Umfangsfläche des Spritzenkörpers in einem Bereich, welcher einem Öffnungswinkel von höchstens 180° beträgt, so dass das Fingerflanschelement besonders einfach auf die Umfangsfläche aufgeschoben werden kann. Daher kann auch eine Ausführungsform, bei der der Öffnungswinkel weniger als 180° und bevorzugt weniger als 175° beträgt, sehr günstig sein, da auf diese Weise sehr einfach das Fingerflanschelement formschlüssig mit dem proximalen Ende des Spritzenkörpers verbunden werden kann.

In einer besonders günstigen Ausführungsform ist ein zweites Fingerflanschelement vorgesehen, welches weitgehend komplementär zu dem ersten Fingerflansch ausgebildet ist. Nach Montage des ersten Fingerflanschelements kann dann besonders einfach mittels einer Drehbewegung der Fingerflansch geschlossen werden und die Umfangsfläche des Spritzenkörpers formschlüssig nahezu vollständig von dem Fingerflansch umschlossen sein. Die Drehachse der Fingerflanschelemente kann bei dieser Ausführungsform parallel zur Mittenachse des Spritzenkörpers angeordnet sein.

Der erfindungsgemäße Fingerflansch kann weitere Fingerflanschelemente umfassen, die ebenso beweglich, bevorzugt drehbeweglich, mit zumindest einem weiteren Fingerflanschelement verbunden sein können. Bevorzugt sind diese weiteren Fingerflanschelemente stoffschlüssig miteinander verbunden.

In einer weiteren günstigen Ausführungsform kann der Fingerflansch beispielsweise drei Fingerflanschelemente umfassen, welche drehbeweglich zueinander mittels zweier Foliengelenke miteinander verbunden sind und welche jeweils eine Ausnehmung aufweisen mit einer Innenkontur, die passgenau gegengleich zu der Umfangsfläche des Spritzenkörpers ausgebildet ist. Dabei kann in einer bevorzugten Ausführungsform jedes Fingerflanschelement die Umfangsfläche des Spritzenkörpers in einem Bereich umfassen, welcher einem Öffnungswinkel von annähernd 60° beträgt, so dass sämtliche Fingerflanschelemente besonders einfach auf die Umfangsfläche des Spritzenkörpers aufgesetzt werden können und durch entsprechende Drehbewegungen die einzelnen Fingerflanschelemente in eine geschlossene Position gebracht werden können, so dass im geschlossenen Zustand der Fingerflansch den Spritzenkörper vollständig formschlüssig umschließt.

Im Sinne der Erfindung ist ferner vorgesehen, dass die Fingerflanschelemente in geschlossener Position miteinander und/oder mit dem Spritzenkörper derart verbunden sind, dass ein ungewolltes Lösen sicher verhindert wird. Hierzu kann eine Verriegelung oder Verrastung der Fingerflanschelemente vorgesehen sein. In anderen Worten, die Fingerflanschelemente können in geschlossener Position miteinander beispielsweise fest verriegelt werden, um ein Öffnen zu verhindern.

Im Fall von zwei Fingerflanschelementen kann beispielsweise die Verriegelung nach der Montage automatisch durch eine Drehbewegung um die gemeinsame Drehachse erfolgen. Hierzu kann beispielsweise an einem ersten Fingerflanschelement ein Verriegelungselement vorgesehen sein, welches in ein komplementäres Aufnahmeelement eines zweiten Fingerflanschelementes eingreift und auf diese Weise eine formschlüssige, dauerhafte Verbindung schafft, welche zugleich ein ungewolltes Lösen verhindert.

Ebenso ist es auch möglich, dass die Fingerflanschelemente in geschlossener Position miteinander und/oder mit dem Spritzenkörper stoffschlüssig verbunden werden. Beispielsweise können die Fingerflanschelemente miteinander und/oder mit dem Spritzenkörper hierzu verklebt sein. Ein ungewolltes Entfernen des montierten Fingerflansches kann so unterbunden werden. Eine stoffschlüssige Verbindung bietet zudem den Vorteil, dass zugleich auch eine Sicherung des Fingerflansches gegen ein Verdrehen um die Mittenachse des Spritzenkörpers bewirkt wird.

In einer Weiterbildung der Erfindung kann die Ausnehmung des Fingerflansches in dem Kontaktbereich mit dem Vorsprung elastisch ausgebildet sein, um einen Toleranzausgleich gegenüber dem Spritzenkörper zu schaffen. Bevorzugt weist die Ausnehmung dabei ein gewisses Übermaß in dem Kontaktbereich auf, so dass im montierten Zustand zusätzlich zu dem Formschluss ein Kraftschluss bzw. eine Klemmung bewirkt wird, der einer Drehbewegung des Fingerflansches um den Spritzenkörper entgegenwirkt. Somit kann einer Drehbewegung des montierten Fingerflansches um die Mittenachse des Spritzenkörpers entgegenwirkt und eine Verdrehung des Fingerflansches verhindert werden.

Der Fingerflansch kann dazu beispielsweise zumindest teilweise eine Nut aufweisen, welche dazu ausgebildet ist, den Vorsprung des Spritzenkörpers aufzunehmen und/oder zu umschließen. Diese Nut kann elastisch ausgebildet sein. Durch ein Übermaß der Nut kann eine Klemmung des Vorsprungs im montierten Zustand erreicht werden.

Durch eine elastische Ausbildung des Fingerflansches im Kontaktbereich mit dem Spritzenkörper lassen sich auch Form- und Lageabweichungen des Spritzenkörpers und/oder des Vorsprungs vorteilhaft kompensieren. Dies ist besonders günstig, wenn der Spritzenkörper aus Glas hergestellt ist und der Vorsprung durch ein Heiß-Umformverfahren erzeugt wird. Hierbei sind gewisse geometrische Abweichungen des Spritzenkörpers häufig nicht ganz auszuschließen.

In einer weiterhin bevorzugten Ausführungsform kann ein Fingerflanschelement, insbesondere zur Erleichterung der Montage auf der Stirnseite des proximalen Endes des Spritzenkörpers, eine Zentrierhilfe umfassen. Diese kann beispielsweise passgenau gegengleich zu der Innenkontur der Öffnung an dem proximalen Ende des Spritzenkörpers ausgebildet sein.

Die Erfindung umfasst ferner einen einstückigen Fingerflansch mit zumindest einem ersten und einem zweiten Fingerflanschelement zur Montage an einem proximalen Ende eines Spritzenkörpers. Dabei sind die einzelnen Fingerflanschelemente beweglich miteinander verbunden, bevorzugt drehbeweglich. Hierzu kann zumindest ein Gelenk, bevorzugt ein Folien- oder Filmgelenk, vorgesehen sein. Ein Fingerflansch kann auch mehr als zwei Fingerflanschelemente umfassen.

Ein erfindungsgemäßer Fingerflansch kann nachträglich an einen Spritzenkörper montiert werden. Bevorzugt ist er dabei mit seiner Innenkontur an die Umfangsfläche des Spritzenkörpers an seinem proximalen Ende, insbesondere an die Außenkontur des Vorsprungs, angepasst. Auf diese Weise ist es möglich, Spritzen mit einem erfindungsgemäßen Fingerflansch nachzurüsten bzw. mit einer Fingerauflagefläche auszustatten.

Ein Fingerflansch im Sinne der Erfindung ist zum einen sehr einfach an der Spritze zu montieren, so dass bezüglich der Montage eine größtmögliche Flexibilität gegeben ist. Darüber hinaus ist er aber auch einfach herzustellen, beispielsweise als einstückiges Spritzgussteil. Hierdurch sind auch die Anforderungen an Transport, Lagerhaltung und Logistik entsprechend gering.

Im montierten Zustand zeichnet sich der Fingerflansch ferner durch einen Schutz gegen ein Demontieren aus. Zudem kann er auch durch die zusätzliche Nut verdrehsicher ausgebildet sein, so dass ein fester Sitz auf der Spritze gewährleistet ist. Er kann überdies auch einen Toleranzausgleich gegenüber Abweichungen der Form oder Lage des Spritzenkörpers umfassen.

### Die Zeichnungen zeigen:

- Fig. 1: schematisch eine beispielhafte Spritze mit einem Fingerflansch im montierten Zustand im Schnitt,
- Fig. 2: schematisch eine beispielhafte Spritze mit einem Fingerflansch im montierten Zustand in einer Seitenansicht,
- Fig. 3: schematisch eine Ausführungsform eines Fingerflansches in einer Draufsicht,
- Fig. 4: schematisch eine weitere Ausführungsform eines Fingerflansches in einer perspektivischen Schrägansicht
- Fig. 5: schematisch die weitere Ausführungsform eines Fingerflansches in einer Draufsicht und
- Fig. 6: schematisch eine Backstop-Funktion.

### Detaillierte Beschreibung bevorzugter Ausführungsformen

Bei der nachfolgenden detaillierten Beschreibung bevorzugter Ausführungsformen bezeichnen um der Klarheit willen gleiche Bezugszeichen im Wesentlichen gleiche Teile in oder an diesen Ausführungsformen.

Es ist dem Fachmann ersichtlich, dass die Erfindung nicht auf die vorstehend anhand der Figuren beschriebenen lediglich beispielhaften Ausführungsformen beschränkt ist, sondern in vielfältiger Weise im Rahmen des Gegenstandes der Patentansprüche variiert werden kann. Insbesondere können auch die Merkmale einzelner Ausführungsbeispiele miteinander kombiniert werden. Die Abmessungen und Größenverhältnisse in den Figuren können aufgrund einer besseren Veranschaulichung von den tatsächlichen Größen abweichen.

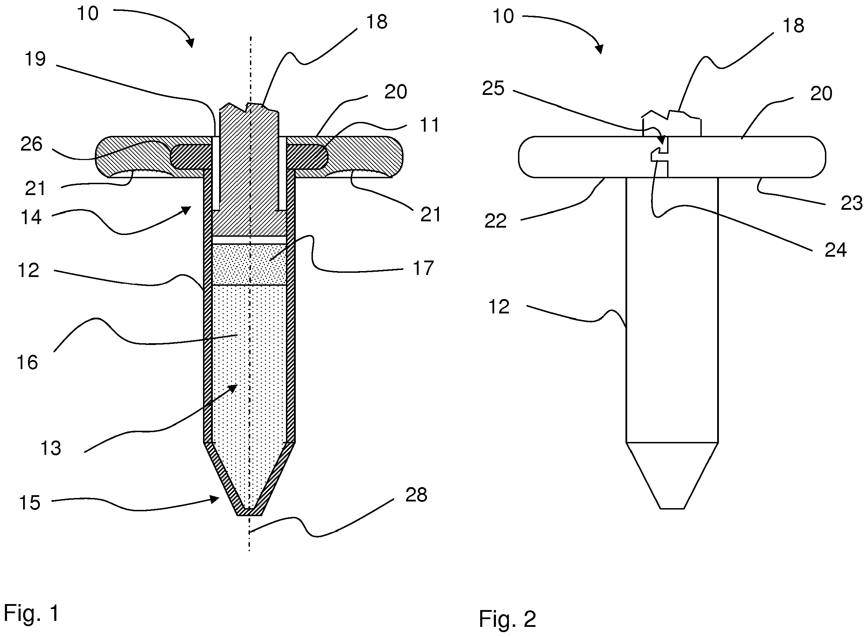

Fig. 1 zeigt schematisch eine beispielhafte Spritze 10 mit einem Fingerflansch 20 im montierten Zustand, also in einer geschlossenen Position, im Schnitt. Die Spritze 10 umfasst eine zylinderförmige Kammer 13 zur Aufnahme einer flüssigen Substanz. Sie kann ungefüllt sein, aber auch bereits mit einer Flüssigkeit gefüllt hergestellt werden. Im Beispiel ist die Spritze 10 mit einer Flüssigkeit 16 gefüllt.

Die Spritze 10 umfasst ferner ein distales Ende 15. In diesem Bereich kann die Spritze mit einer Nadel oder einer Nadelaufnahmevorrichtung verbunden werden (nicht dargestellt), um eine in der Kammer 13 vorhandene Flüssigkeit 16 auszutreiben. Die distale Spitze der Spritze 10 kann hierzu einen Durchlass aufweisen. Es kann auch durch das Verbinden der Spritze 10 mit einer Nadel ein Durchlass geschaffen werden, durch die die Flüssigkeit 16 ausgetrieben werden kann.

Die Spritze 10 umfasst weiterhin ein proximales Ende 14, welches stirnseitig mit einem umlaufenden Vorsprung, im Beispiel mit einem ringförmig umlaufenden, über die Mantelfläche des Spritzenkörpers vorstehenden Vorsprung 11, ausgebildet ist. Der Vorsprung 11 kann umlaufend ausgebildet sein, oder beispielsweise nur auf einer Seite des Spritzenkörpers angeordnet sein. Stirnseitig weist die Spritze 10 eine innere Öffnung 19 auf, durch die eine Kolbenstange 18 in die Kammer 13 eingeführt werden kann. Im gefüllten Zustand ist die Spritze 10 mit einem Stopfen 17 ausgestattet, welcher die Kammer 13 fluiddicht gegen ein ungewolltes Austreten der Substanz 16 abdichtet. Die Kolbenstange 18 kann in die Kammer 13 eingeführt und mit dem Stopfen 17 verbunden werden, beispielsweise durch Eindrehen in eine im Stopfen vorgefertigte Gewindebohrung. Auf diese Weise kann mittels der Kolbenstange 18 der Stopfen 17 axial in der Kammer 13 bewegt werden und durch eine Hubbewegung in Richtung des distalen Endes die Flüssigkeit 16 ausgetrieben werden. Im Fall einer ungefüllten Spritze 10 kann der Kolben aber auch derart ausgebildet sein, dass er fluiddicht mit der Innenwand der Kammer 13 abschließt. Durch eine Hubbewegung in Richtung des proximalen Endes 14 kann dann durch das distale Ende eine Flüssigkeit in die Kammer 13 eingezogen und durch eine umgekehrte Hubbewegung wieder ausgetrieben werden.

Um die Handhabung einer derartigen Spritze 10 zu erleichtern, ist eine Fingerauflage hilfreich, deren Verwendung als hinreichend bekannt angenommen wird, so dass auf eine ausführliche Beschreibung an dieser Stelle verzichtet wird.

Erfindungsgemäß wird ein Fingerflansch 20 mit zumindest einer Fingerauflage 21 zur Verbindung mit einer Spritze 10 vorgeschlagen. Der Fingerflansch 20 ist dabei in geschlossener Position mit dem Vorsprung 11 des Spritzenkörpers 12 formschlüssig verbunden, wobei zumindest eine gemeinsame Kontaktfläche zwischen dem Fingerflansch 20 und dem Vorsprung 11 ausgebildet wird. Auf diese Weise kann ein axiales Verschieben des Fingerflansches 20 relativ zu dem Spritzenkörper 12 verhindert werden.

Im abgebildeten Beispiel umfasst der Fingerflansch 20 zwei Fingerauflageflächen 21, welche gegenüberliegend angeordnet sind und radial nach außen über den Spritzenkörper 12 hervorstehen. Hiermit kann eine besondere einfache und sichere Handhabung der Spritze 10 gewährleistet werden.

Die Innenkontur des Fingerflansches 20 ist derart ausgebildet, dass sie passgenau gegengleich zur Außenkontur des Spritzenkörpers 12, insbesondere des Vorsprungs 11, ausgebildet ist. In anderen Worten, die Innenkontur des Fingerflansches 12 ist der Außenkontur des umlaufenden ringförmigen Vorsprungs 11 des Spritzenkörpers derart angepasst, dass in geschlossener Position eine formschlüssige Verbindung geschaffen werden kann. Im montierten Zustand umgibt der Fingerflansch den umlaufenden ringförmigen Vorsprung zumindest teilweise. Dabei umfasst der Fingerflansch 20 eine Ausnehmung, im Beispiel eine durchgehende Öffnung, zur Durchführung der Kolbenstange, welche im Innendurchmesser auf die Öffnung 19 der Spritze 10 abgestimmt ist.

Der Fingerflansch 20 ist im Sinne der Erfindung einstückig ausgebildet, um eine besonders einfache Herstellbarkeit zu gewährleisten. Dabei kann er vollständig oder nur in bestimmten Bereichen ein elastisches Material umfassen. Das elastische Material kann ein thermoplastischer Kunststoff sein, welcher sich durch ein geringes Gewicht und eine einfach Formbarkeit auszeichnet. Für das Filmscharnier und/oder den Kontaktbereich zwischen Fingerflansch 20 und Spritzenkörper 12 können die bekannten, gängigen Materialien verwendet werden, umfassend PP, PE, POM, PBT, PA, PES, TPE, aber auch beispielsweise glasfaserverstärkte Kunststoffe. Somit sind auch besonders kostengünstige Spritzgussverfahren für die Herstellung des Fingerflansches 20 möglich.

In geschlossener Position umschließt der Fingerflansch 20 den zumindest einen Vorsprung 11, so dass eine formschlüssige Verbindung geschaffen werden kann. Diese formschlüssige Verbindung verhindert eine axiale Bewegung des Fingerflansches 20 entlang der Mittenlinie 28 des Spritzenkörpers 12, so dass eine sehr stabile und sichere Fingerauflagemöglichkeit 21 geschaffen werden kann.

Zusätzlich kann der Fingerflansch 20 im Bereich der Kontaktfläche zu dem Spritzenkörper 12 auch mit diesem stoffschlüssig, etwa mittels einer Klebung, verbunden werden, um eine noch größere Stabilität der Verbindung zu schaffen und obendrein ein ungewolltes Lösen des Fingerflansches auszuschließen. Auch eine Sicherung gegen ein Verdrehen des Fingerflansches relativ gegenüber dem Spritzenkörper kann hierdurch realisiert werden.

Im Bereich des Vorsprungs 11 kann zumindest ein Fingerflanschelement zusätzlich zumindest teilweise eine Nut 26 aufweisen, welche zumindest teilweise passgenau gegengleich zu dem aufzunehmenden Vorsprung 11 ausgebildet ist und entlang der Ausnehmung verläuft. Diese kann mit einem kleinen Untermaß gegenüber dem Vorsprung gefertigt werden, so dass bei der Montage zusätzlich eine Klemmung des Vorsprungs 11 bewirkt wird. Auf diese Weise kann sehr einfach eine Sicherung gegen ein Verdrehen des Fingerflansches 20 um die Mittenlinie 28 gewährleistet werden.

In Fig. 2 ist schematisch eine beispielhafte Spritze 10 mit einem Fingerflansch 20 im montierten Zustand in einer Seitenansicht abgebildet. Der abgebildete Fingerflansch 20 umfasst zwei im Wesentlichen komplementär zueinander ausgebildete Fingerflanschelemente 22, 23. Beide Fingerflanschelemente 22, 23 weisen eine Ausnehmung auf mit einer Innenkontur, die passgenau gegengleich zu der Außenkontur des Vorsprungs 11 der Spritze 10 ist. Im montierten Zustand umgreifen beide Fingerflanschelemente 22, 23 die Umfangsfläche des Spritzenkörpers 12 an seinem proximalen Ende 14 vollständig.

Die Ausnehmungen sind in axialer Richtung im Wesentlichen U-förmig ausgebildet, um den umlaufenden Vorsprung 11 optimal umgreifen zu können. Die Ausnehmungen sind dabei besonders vorteilhaft derart angeordnet, dass diese im montierten Zustand eine durchgehende Öffnung bilden, welche mit der Öffnung 19 des Spritzenkörpers 12 übereinstimmt und so die Durchführung der Kolbenstange 18 ermöglicht.

Der Fingerflansch 20 umfasst ferner ein Gelenk zum drehbeweglichen Verbinden der beiden Fingerflanschelemente 22, 23, welches rückseitig angeordnet und von daher in der Abbildung nicht erkennbar ist, sowie gegenüberliegend Verriegelungselemente zum Verrasten oder Verriegeln der beiden Fingerflanschelemente in geschlossener Position. In der geschlossenen Position kann ein Verriegelungselement 24 des ersten Fingerflanschelementes 22 in ein passgenau gegengleich ausgebildetes Aufnahmeelement 25 eines zweiten Fingerflanschelements 23 eingreifen, um im montierten Zustand die beiden Fingerflanschelemente miteinander unlösbar verriegeln zu können.

Das Verriegelungs- bzw. Rastelement 24 ist lediglich schematisch und beispielhaft dargestellt. Besonders günstig es derart ausgebildet, dass ein Einrasten, bevorzugt während der Drehbewegung zum Schließen der Fingerflanschelemente, ermöglicht wird, und wobei gleichzeitig einem erneuten Öffnen durch die Ausgestaltung der Rastelemente entgegengewirkt wird. Somit wird ein ungewolltes Öffnen und Lösen des Fingerflansches 20 von der Spritze 10 unterbunden.

Hierdurch kann daher eine unlösbare Verbindung der beiden Fingerflanschelemente 22, 23 erfolgen. Ein erstes Fingerflanschelement 23 kann dabei beispielsweise mit einem federelastischen Widerhaken ausgebildet sein, welcher in eine passgenau gegengleiche Aufnahme 25 eines weiteren Fingerflanschelements 22 eingreifen kann.

Der federelastische Widerhaken sowie die gegengleiche Aufnahme können dabei derart ausgebildet sein, dass ein erneutes Öffnen ohne Zerstörung der Verbindungselemente nicht möglich ist. Auf diese Weise kann der Fingerflansch 20 formschlüssig und unlösbar mit der Spritze 10 verbunden werden. Einem unbeabsichtigten Entfernen des Fingerflansches 20 von dem Spritzenkörper 12 kann auf diese Weise entgegengewirkt werden.

Der Fingerflansch ist einstückig ausgebildet. Er kann dabei zwei oder mehr Fingerflanschelemente umfassen, welche beweglich zueinander, bevorzugt drehbeweglich, angeordnet sind und stofflich fest miteinander verbunden sind.

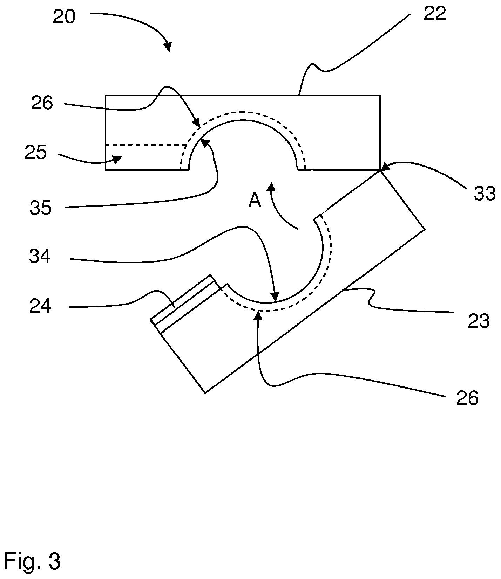

Fig. 3 zeigt schematisch eine beispielhafte Ausführungsform eines Fingerflansches 20 in einer Draufsicht. Die Abbildung zeigt einen Fingerflansch 20 in montagebereiter Form, also vor der Montage an dem Spritzenkörper 12. Die zwei Fingerflanschelemente 22, 23 sind also in einer geöffneten Position gezeigt. Herstellbedingt kann die geöffnete Position selbstverständlich von der abgebildete Form abweichen.

Die beiden Fingerflanschelemente 22, 23 sind weitgehend komplementär zueinander ausgebildet. Beide Fingerflanschelemente sind jeweils entlang einer gemeinsamen geraden Kante stoffschlüssig und drehbeweglich miteinander verbunden über ein Gelenk 33. Das Gelenk 33 kann ein Filmscharnier, ein Folien- oder ein Filmgelenk umfassen, welches eine Drehbewegung der Fingerflanschelemente 22, 23 relativ zueinander erlaubt und eine Drehachse festlegt, welche im abgebildeten Beispiel entlang der gemeinsamen Kante verläuft.

Im abgebildeten Beispiel liegt die Drehachse des Fingerflansches 20 parallel zur Mittenlinie 28 des Spritzenkörpers 12, wenn der Fingerflansch 20 an der Spritze 10 fertig montiert ist. Die Lage der Drehachse relativ zur Mittenlinie 28 des Spritzenkörpers bestimmt die Art und Weise, wie der Fingerflansch an dem Spritzenkörper montiert und sodann geschlossen werden kann. In weiteren Ausführungsformen kann die Drehachse dabei auch senkrecht zur Mittenachse 28 des Spritzenkörpers 12 angeordnet sein.

Der Fingerflansch 20 ist mit einer zentralen Durchgangsöffnung ausgebildet, welche sich durch die Form zweier gleich großer Ausnehmungen 34, 35 in beiden Fingerflanschelementen 22, 33 ergibt. Die Ausnehmungen 34, 35 sind dabei von annähernd U-förmiger Form und der Außenfläche des Spritzenkörpers angepasst. Hierdurch kann im montierten Zustand zumindest ein Abschnitt des Spritzenkörpers durch den Fingerflansch derart umschlossen werden, dass der Spritzenkörper 12 in einem Umfangsbereich, der einem Öffnungswinkel von mehr als 180° entspricht, umschlossen wird. Im vorliegenden Beispiel kann der ringförmige Vorsprung 11 des Spritzenkörpers 12 durch den Fingerflansch, welcher hierzu mit einer Nut 26 ausgebildet ist, nahezu vollständig umschlossen werden, was einem Winkelbereich von gleich oder nahezu 360° entspricht. Dabei umgreift ein einzelnes Fingerflanschelement 22, 23 den ringförmigen Vorsprung jeweils nur zu einem Teil, vorliegend in einem Umfangsbereich, der einem Öffnungswinkel von etwa 180° entspricht.

Es sind selbstverständlich auch andere Öffnungswinkel möglich. Bei einem Fingerflansch 20 mit beispielsweise drei komplementär zueinander ausgebildeten Fingerflanschelementen kann jedes einzelne Fingerflanschelement den Spritzenkörper 12 beispielsweise in einem Umfangsbereich umfassen, welcher einem Öffnungswinkel von 120° entspricht. Im geschossenen Zustand kann aufgrund der drei einzelnen Fingerflanschelemente der Fingerflansch den ringförmigen Vorsprung dann zu 360° umfassen, so dass eine besonders stabile, formschlüssige Verbindung geschaffen werden kann.

Auf diese Weise ist eine besonders einfache Montage des Fingerflansches 20 an dem Spritzenkörper 12 möglich, da keine zusätzliche Kraftkomponente zur Überwindung der Haftreibung durch axiales Aufschieben oder zum Aufdrücken des Fingerflanschelements erforderlich ist.

Die in axialer Richtung U-förmige Ausnehmung 34, 35 ermöglicht es, einen Umfangsabschnitt des Spritzenkörpers 12 zumindest teilweise zu umschließen.

Zumindest eine Ausnehmung kann dabei in einer Weiterbildung der Erfindung besonders günstig mit einer nach Innen verlaufenden, mittig angeordneten Nut 26 ausgebildet sein, welche mit leichtem Untermaß passgenau gegengleich zur Außenkontur des Vorsprungs 11 derart ausgebildet ist, dass zusätzlich zu dem Formschluss auch eine Klemmung des Vorsprungs bewirkt wird. Dabei ist es von Vorteil, wenn das Material des Fingerflanschelements 22, 23, zumindest in dem Kontaktbereich mit dem Vorsprung 11, eine Elastizität aufweist, so dass geringfügige Form- und Lageabweichungen des Vorsprungs 11, aber auch in der Außenkontur oder in dem Außendurchmesser des Spritzenkörpers 12, kompensiert werden können.

Die Ausnehmung 34, 35 des Fingerflansches 20 ist vorteilhaft derart angeordnet, dass sie im montierten Zustand deckungsgleich mit der Öffnung 19 des Spritzenkörpers 12 ist und auf diese Weise eine Durchgangsöffnung schafft. Auf diese Weise kann die Kolbenstange 18 durch die Durchgangsöffnung des Fingerflansches 20 in die Kammer 16 des Spritzenkörpers 12 geführt und axial bewegt werden.

Das Verriegelungselement 24 sowie das Aufnahmeelement 25 können in einer günstigen Ausführungsform auf einer von der gemeinsamen geraden Kante abgewandten oder gegenüberliegenden Seite angeordnet sein. Im abgebildeten Beispiel ist ein elastisches Federelement 24 des Fingerflanschelements 23 zum Einrasten in die entsprechend ausgebildete Aufnahme 25 des Fingerflanschelements 22 auf den im montierten Zustand zusammenstoßende Flächen der beiden Fingerflanschelemente 22, 23 angeordnet.

Eine Montage des Fingerflansches 20 an dem Spritzenkörper 12 kann beispielsweise derart erfolgen, dass zunächst ein erstes Fingerflanschelement 22 auf den Spritzenkörper 12 aufgeschoben oder aufgesteckt und sodann durch eine Drehbewegung in Richtung A der Fingerflansch geschlossen werden kann. Hierdurch kann ein Formschluss zwischen Fingerflansch 20 und Vorsprung 11 geschaffen werden, welcher sehr stabil ist.

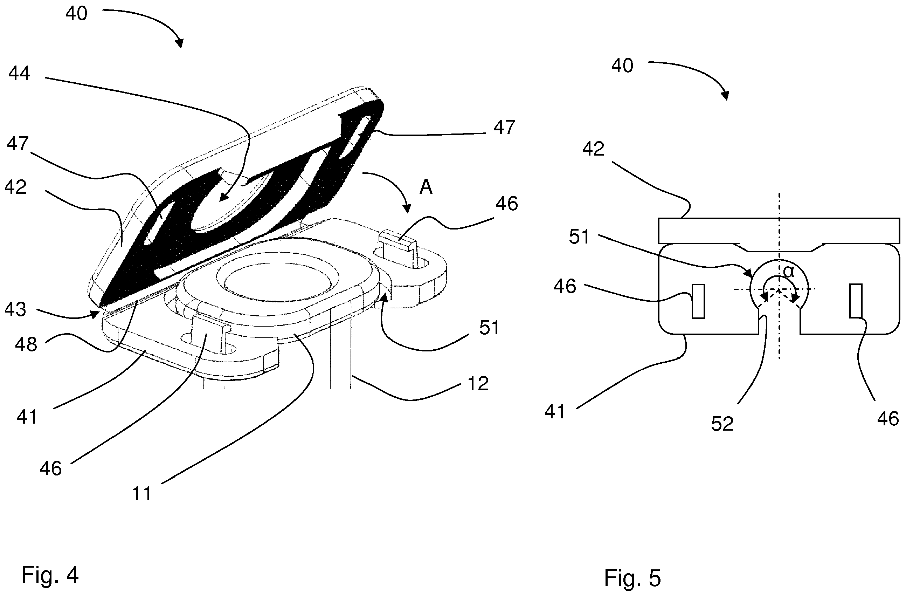

Eine weitere bevorzugte Ausführungsform eines erfindungsgemäßen Fingerflansches zeigt Fig. 4 schematisch in einer perspektivischen Schrägansicht.

Der abgebildete Fingerflansch 40 umfasst zwei Fingerflanschelemente 41, 42, welche entlang einer gemeinsamen geraden Kante 48 aneinanderstoßen und stoffschlüssig und beweglich zueinander verbunden sind. Entlang der gemeinsamen geraden Kante 48 ist ein Gelenk, im Beispiel ein Filmgelenk 43, angeordnet, so dass beide Fingerflanschelemente 41, 42 um die durch das Gelenk ausgebildete Drehachse zueinander von einer geöffneten in eine geschlossene Position gedreht werden können. Im abgebildeten Beispiel liegt diese Drehachse senkrecht zur Mittenlinie 28 des Spritzenkörpers 12, wenn der Fingerflansch 40 an der Spritze 10 montiert ist.

Bei dieser Ausführungsform kommt eine Kontaktfläche zwischen Fingerflansch 40 und Spritzenkörper 12 zustande, welche sowohl einen Teil der Mantelfläche, insbesondere im Bereich des Vorsprungs, als auch zumindest einen Teil der Stirnseite des Spritzenkörpers umfasst. Zumindest ein Fingerflanschelement 42 ist daher passgenau gegengleich zu der Stirnseite des Spritzenkörpers ausgebildet.

Ein erstes Fingerflanschelement 41 ist derart ausgebildet, dass es eine Ausnehmung 51 aufweist, die ein radiales Aufschieben auf das proximale Ende des Spritzenkörpers 12 ermöglicht. Dabei wird zwischen dem Vorsprung 11 des Spritzenkörpers und dem Fingerflanschelement 41 eine Kontaktfläche ausgebildet, welche in axialer Richtung etwa hälftig den in Richtung des distalen Endes des Spritzenkörpers weisenden Anteil des Vorsprungs 11 umfasst. Die Ausnehmung 51 ist daher passgenau gegengleich zu der Außenkontur desjenigen Teilabschnitts des Vorsprungs ausgebildet, welcher in Richtung des distalen Endes des Spritzenkörpers weist. Im montierten Zustand umgreift das Fingerflanschelement 41 die Mantelfläche des Spritzenkörpers 12 in einem Bereich, der einem Öffnungswinkel von 180° oder mehr entspricht. Sofern der Öffnungswinkel mehr als 180° entspricht, ist eine gewisse Elastizität des Fingerflanschelements 41 günstig, um ein seitliches Aufschieben auf den Spritzenkörper 12 zu ermöglichen.

Eine weitere Kontaktfläche wird zwischen dem weiteren Fingerflanschelement 42 und dem in Richtung der Öffnung des Spritzenkörpers 12 weisenden Vorsprung und/oder zumindest einem Teil der Stirnseite des Spritzenkörpers 12 gebildet, wenn nach dem Aufschieben des Fingerflanschelements 41 das weitere Fingerflanschelement 42 geschlossen wird. Zum Schließen der beiden Fingerflanschelemente kann eine Bewegung in Drehrichtung A erfolgen.

Im Beispiel ist das zuerst montierbare Fingerflanschelement 41 mit zwei in Richtung der Öffnung des Spritzenkörpers vorspringenden elastischen Federelementen 46 ausgebildet, welche beim Schließen in die passgenau gegengleich ausgebildeten Aufnahmen 47 des anderen Fingerflanschelements 42 eingreifen. Hierdurch können beide Fingerflanschelemente sicher miteinander verrastet werden, wobei durch entsprechende Ausgestaltung ein erneutes Öffnen verhindert werden kann.

Der Fingerflansch 40 umfasst ferner eine Durchgangsöffnung, welche in dem Fingerflanschelement 42 als mittige, durchgehende Öffnung 44 ausgebildet ist, und welche in dem Fingerflanschelement 41 einen Teil der Ausnehmung umfasst.

Die im montierten Zustand gebildete Kontaktfläche zwischen Fingerflansch 20, 40 und Spritze 10 kann also einen Teil der Umfangsfläche des Spritzenkörpers, insbesondere an seinem proximalen Ende 14, und/oder einen Teil der Stirnseite des Spritzenkörpers 12 umfassen.

In Fig. 5 ist schematisch ein Fingerflansch 40 in einer Draufsicht in einem für die Montage geeigneten, geöffneten Zustand abgebildet. Die abgebildete, annähernd U-förmige Ausnehmung 51 ist mittig ausgebildet zur Durchführung der Kolbenstange und verläuft zur Außenkante hin in zwei sich gegenüberliegenden, kragenförmig vorstehenden Abschnitten 52. Bei dieser Ausführungsform wird die Kolbenstange oder die Kolbenstange mit einem Stopfen erst nach dem Anbringen und Schließen des Fingerflansches 40 montiert, um durch die Durchgangsöffnung 44 geschoben werden zu können. Eine Backstop-Funktion kann durch eine Ausbildung des Fingerflansches im Bereich der Durchgangsöffnung 44 mit einem elastischen Material geschaffen werden, so dass sich die Durchgangsöffnung 44 elastisch aufweiten kann bei der Montage der Kolbenstange oder der Kolbenstange mit einem Stopfen.

Durch diese Form der Ausnehmung 51 kann ein Umgreifen des proximalen Endes des Spritzenkörpers erreicht werden, welches einem Öffnungswinkel α von mehr als 180° entspricht. Im abgebildeten Beispiel hat der Öffnungswinkel α eine Größe von etwa 220°. Zumindest das Fingerflanschelement 41 ist dabei, insbesondere im Bereich der Ausnehmung, elastisch ausgebildet, so dass ein seitliches Aufschieben auf den Spritzenkörper 12 erleichtert wird. Die Ausnehmung des Fingerflanschelements kann aber auch derart ausgebildet sein, dass ein Umgreifen mit einem Öffnungswinkel von weniger als 180° erreicht wird. Hierdurch kann mit geringer Kraftaufwendung die Montage des Fingerflanschelements erfolgen.

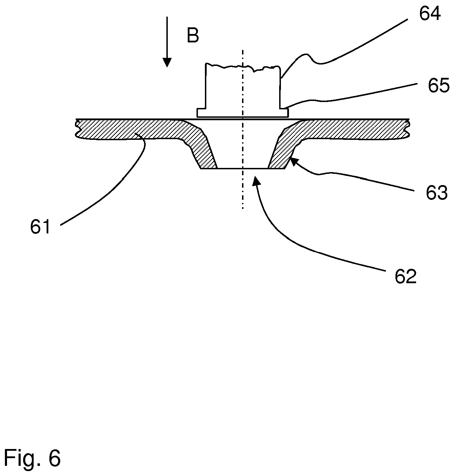

In Fig. 6 ist schließlich schematisch eine Backstop-Funktion dargestellt. Ein ausschnittsweise dargestelltes Fingerflanschelement 61 aus einem elastischen Material umfasst eine Durchgangsöffnung 62. Umlaufend um die Durchgangsöffnung 62 ist das Fingerflanschelement mit einem kragenförmigen Vorsprung 63 ausgebildet, welcher sich entgegen der Montagerichtung des Stopfens oder der Kolbenstange verjüngt.

Sofern nun ein ausschnittsweise dargestellter Stopfen 64 in Montagerichtung B durch die Durchgangsöffnung 62 geschoben wird, dehnt sich das elastische Material des Fingerflanschelements, bevorzugt im Bereich des kragenförmigen Vorsprungs 63. Durch diese Aufweitung der Durchgangsöffnung 62 kann der Stopfen 64 hindurchgeschoben werden. Nach erfolgtem Durchschieben des Stopfens 64 verringert sich die Aufweitung der Durchgangsöffnung 62 wieder und ein weiterer Vorsprung 65 des Stopfens 64 verhindert eine Bewegung entgegen der Montagerichtung B. In gleicher Weise kann auch eine Backstop-Funktion für eine Kolbenstange (nicht dargestellt) realisiert werden.

Die Erfindung umfasst ferner einen einstückigen Fingerflansch 20, 40, der ausgebildet ist, um mit einem Spritzenkörper 12 an seinem proximalen Ende 14, insbesondere im Bereich des Vorsprungs 11, verbunden werden zu können. Der Fingerflansch kann zwei oder mehr Fingerflanschelemente 22, 23, 41, 42 umfassen, welche beweglich, bevorzugt drehbeweglich, miteinander verbunden sind. In einer bevorzugten Ausführungsform sind die Fingerflanschelemente stoffschlüssig miteinander verbunden.

Die stoffschlüssige drehbewegliche Verbindung der Fingerflanschelemente kann dabei durch ein Gelenk, bevorzugt ein Filmscharnier, ein Folien- oder ein Filmgelenk, vorgesehen sein.

Ein erfindungsgemäßer Fingerflansch 20, 40 kann an einer Spritze 10 montiert werden. Bevorzugt ist dabei die Innenkontur des Fingerflansches passgenau gegengleich zu der Außenkontur des Spritzenkörpers 12 an seinem proximalen Ende 14 ausgebildet, um eine kraft- und/oder stoffschlüssige Verbindung zu schaffen. Bei einem Spritzenkörper mit einer Nut an seinem proximalen Ende kann der Fingerflansch demnach auch einen passgenau gegengleich ausgebildeten Vorsprung aufweisen.

Auf diese Weise ist es möglich, Spritzen mit einem Fingerflansch nachzurüsten und mit einer Fingerauflagefläche auszustatten.

Der erfindungsgemäße Fingerflansch 20, 40 ist zum einen sehr einfach an dem Spritzenkörper zu montieren. Darüber hinaus ist er aber auch kostengünstig und in hohen Stückzahlen herzustellen, beispielsweise als einstückiges Spritzgussteil oder aber in einem Mehrfachkomponenten-Spritzgussverfahren oder einem In-mould-Verfahren.

Im montierten Zustand zeichnet sich der Fingerflansch ferner durch einen Schutz gegen ein Demontieren aus. Hierzu kann die Verrastung ausgebildet sein, um eine unlösbare Verriegelung in geschlossener Position der Fingerflanschelemente zu erreichen. Zudem kann der Fingerflansch auch drehsicher befestigt sein, so dass ein fester Sitz auf der Spritze gewährleistet ist. Er kann überdies auch einen Toleranzausgleich bereitstellen.

### Bezugszeichenliste

- 10: Spritze
- 11: Vorsprung
- 12: Spritzenkörper
- 13: Kammer
- 14: proximales Ende
- 15: distales Ende
- 16: Flüssigkeit
- 17: Stopfen
- 18: Kolbenstange
- 19: innere Öffnung
- 20: Fingerflansch
- 21: Fingerauflage
- 22: Fingerflanschelement
- 23: Fingerflanschelement
- 24: Verriegelungselement
- 25: Aufnahmeelement
- 26: Nut
- 28: Mittenlinie
- 33: Gelenk
- 34: Ausnehmung
- 35: Ausnehmung
- 40: Fingerflansch
- 41: Fingerflanschelement
- 42: Fingerflanschelement
- 43: Filmgelenk
- 44: Durchgangsöffnung
- 46: Federelement
- 47: Aufnahme
- 48: Kante
- 51: Ausnehmung
- 52: Abschnitt
- 61: Fingerflanschelement (Ausschnitt)
- 62: Durchgangsöffnung
- 63: kragenförmiger Vorsprung
- 64: Stopfen
- 65: Vorsprung

## Patentansprüche

1. Fingerflansch (20) insbesondere zum kraftschlüssig und/oder formschlüssig Verbinden mit dem proximalen Ende eines Spritzenkörpers (12), mit einer Ausnehmung oder Durchgangsöffnung zur Durchführung einer Kolbenstange, der einstückig ausgebildet ist, umfassend zumindest ein erstes und ein zweites Fingerflanschelement (22, 23, 41, 42), welche beweglich, bevorzugt drehbeweglich, miteinander verbunden sind, wobei eine unlösbare Verriegelung der Fingerflanschelemente vorgesehen ist und wobei der Fingerflansch (20) ausgebildet ist mit einer Auszugsicherung der Kolbenstange (18).

2. Fingerflansch (20) nach vorstehendem Anspruch, **dadurch gekennzeichnet, dass** das zumindest erste und zweite Fingerflanschelement (22, 23, 41, 42) mittels eines Gelenks, bevorzugt stoffschlüssig mittels eines Folien- oder Filmgelenks, miteinander verbunden sind.

3. Fingerflansch (20) nach einem der beiden vorstehenden Ansprüche, **dadurch gekennzeichnet, dass** der Fingerflansch ein Herausziehen und/oder Verdrehen der Kolbenstange und/oder des Stopfens verhindern kann.

4. Spritze (10), umfassend einen zylinderförmigen Spritzenkörper (12) mit einem distalen Ende und einem proximalen Ende sowie einen Fingerflansch (20) mit zumindest einer Auflagefläche für einen Finger nach einem der vorstehenden Ansprüche, wobei
- der Spritzenkörper (12) eine zylinderförmige Kammer umfasst zur Aufnahme einer flüssigen Substanz,
- das proximale Ende des Spritzenkörpers (12) eine Öffnung (19) aufweist, durch die eine Kolbenstange (18) geführt werden kann,
- das proximale Ende des Spritzenkörpers ferner zumindest einen radial nach außen vorspringenden, über die Mantelfläche des Spritzenkörpers vorstehenden Vorsprung (11) umfasst, wobei
- der Fingerflansch (20) einstückig ausgebildet ist,
- der Fingerflansch (20) zumindest ein erstes und ein zweites Fingerflanschelement (22, 23, 41, 42) umfasst, welche beweglich miteinander verbunden sind und von einer geöffneten Position in eine geschlossene Position bewegt werden können,
- der Fingerflansch (20) in geschlossener Position zumindest teilweise mit dem zumindest einen Vorsprung (11) kraftschlüssig verbunden ist und den Vorsprung (11) zumindest teilweise derart umschließt, dass in geschlossener Position ein axiales Verschieben des Fingerflansches (20) relativ zu dem Spritzenkörper (12) verhindert wird, wobei eine unlösbare Verriegelung der Fingerflanschelemente vorgesehen ist, wobei die Fingerflanschelemente nicht zerstörungsfrei voneinander trennbar sind, wobei der Fingerflansch (20) ausgebildet ist mit einer Auszugsicherung der Kolbenstange (18), und wobei
- der Fingerflansch (20) eine Ausnehmung zur Durchführung der Kolbenstange (18) aufweist.

5. Spritze (10) nach vorstehendem Anspruch, **dadurch gekennzeichnet, dass** der Fingerflansch (20) in geschlossener Position zumindest teilweise mit dem zumindest einen Vorsprung (11) formschlüssig verbunden ist.

6. Spritze (10) nach einem der vorstehenden Ansprüche 4 oder 5, **dadurch gekennzeichnet, dass** eine Kolbenstange (18) zur Verbindung mit einem Stopfen (17) in der Kammer oder eine Kolbenstange mit einem Stopfen durch die Öffnungen (19) an dem proximalen Ende des Spritzenkörpers (12) und des Fingerflansches (20) eingeführt werden kann.

7. Spritze (10) nach einem der vorstehenden Ansprüche 4 bis 6, **dadurch gekennzeichnet, dass** das erste und das zweite Fingerflanschelement (22, 23, 41, 42) mittels eines Gelenks (33, 43) drehbeweglich miteinander verbunden sind.

8. Spritze (10) nach einem der vorstehenden Ansprüche 4 bis 7, **dadurch gekennzeichnet, dass** zumindest das erste und das zweite Fingerflanschelement (22, 23, 41, 42) mittels eines Folien- oder Filmgelenks stoffschlüssig miteinander verbunden sind.

9. Spritze (10) nach einem der vorstehenden Ansprüche 4 bis 8, **dadurch gekennzeichnet, dass** die Ausnehmung (34, 35, 51) des Fingerflansches (20) eine Innenkontur aufweist, welche zumindest teilweise passgenau gegengleich zu der Außenkontur des zumindest einen Vorsprungs (11) ausgebildet ist.

10. Spritze (10) nach einem der vorstehenden Ansprüche 4 bis 9, **dadurch gekennzeichnet, dass** die Ausnehmung des Fingerflansches (20) zumindest teilweise ein Übermaß gegenüber dem Vorsprung (11) aufweist.

11. Spritze (10) nach einem der vorstehenden Ansprüche 4 bis 10, **dadurch gekennzeichnet, dass** die Ausnehmung des Fingerflansches (20) in dem Kontaktbereich mit dem Spritzenkörper (12) zumindest teilweise durch ein elastisches Material gebildet wird, um geometrische Abweichungen des Spritzenkörpers (12) zu kompensieren.

12. Spritze (10) nach einem der vorstehenden Ansprüche 4 bis 11, **dadurch gekennzeichnet, dass** zumindest ein Fingerflanschelement die Umfangsfläche des Spritzenkörpers in einem Bereich umschließt, welcher einem Öffnungswinkel α von höchstens 180°, bevorzugt weniger als 180° und bevorzugt weniger als 175° entspricht.

13. Spritze (10) nach einem der vorstehenden Ansprüche 4 bis 12, **dadurch gekennzeichnet, dass** die Fingerflanschelemente in geschlossener Position miteinander verrastet und/oder unlösbar verriegelt sind.

14. Spritze (10) nach einem der vorstehenden Ansprüche 4 bis 13, **dadurch gekennzeichnet, dass** der Fingerflansch (20) ein elastisches Material umfasst, umfassend PP, PE, POM, PBT, PA, PES, TPE, oder glasfaserverstärkte Kunststoffe.

## Claims

1. A finger flange (20), in particular for being frictionally and/or form-fittingly connected to the proximal end of a syringe body (12), having a recess or through-opening for extending therethrough a plunger rod, the finger flange being formed in one piece comprising at least one first and one second finger flange member (22, 23, 41, 42) which are coupled so as to be movable relative to one another, preferably pivotably movable, the finger flange members being adapted for being non-releasably locked, and wherein the finger flange (20) is formed with a pull-out lock for the plunger rod (18).

2. The finger flange (20) according to the preceding claim, **characterised in that** the at least first and second finger flange members (22, 23, 41, 42) are connected to one another through a material bond, by a hinge, preferably a film hinge or living hinge.

3. The finger flange (20) according to any one of the two preceding claims, **characterised in that** the finger flange is able to prevent the plunger rod and/or the stopper from being pulled out and/or twisted.

4. A syringe (10), comprising a cylindrical syringe body (12) having a distal end and a proximal end and a finger flange (20) with at least one support surface for a finger according to any one of the preceding claims, wherein
- the syringe body (12) has a cylindrical cavity for holding a liquid substance;
- the proximal end of the syringe body (12) has an opening (19) through which a plunger rod (18) can be extended;
- the proximal end of the syringe body furthermore comprises at least one projection (11) protruding radially outwards and protruding beyond the lateral surface of the syringe body; wherein
- the finger flange (20) is formed in one piece;
- the finger flange (20) comprises at least one first and one second finger flange member (22, 23, 41, 42) which are coupled so as to be movable relative to one another and can be moved from an open position to a closed position;
- in the closed position, the finger flange (20) is at least in part frictionally connected to the at least one projection (11) and at least partially surrounds the projection (11) such that axial displacement of the finger flange (20) relative to the syringe body (12) is prevented in the closed position, wherein the finger flange members are adapted for being non-releasably locked, wherein the finger flange members are not separable from one another in a nondestructive way, wherein the finger flange (20) is formed with a pull-out lock for the plunger rod (18); and wherein
- the finger flange (20) has a recess for extending therethrough the plunger rod (18).

5. The syringe (10) according to the preceding claim, **characterised in that**, when in the closed position, the finger flange (20) is at least partially connected to the at least one projection in a form-fitting manner.

6. The syringe (10) according to any one of the preceding claims 4 or 5, **characterized in that** a plunger rod (18) for being connected to a stopper (17) inside the cavity or a plunger rod with a stopper can be introduced through the openings (19) at the proximal end of the syringe body (12) and of the finger flange (20).

7. The syringe (10) according to any one of the preceding claims 4 to 6, **characterized in that** the first and second finger flange members (22, 23, 41, 42) are pivotably connected to one another by a hinge (33, 43).

8. The syringe (10) according to any one of the preceding claims 4 to 7, **characterized in that** at least the first and the second finger flange members (22, 23, 41, 42) are connected to one another through a material bond, by a film hinge or living hinge.

9. The syringe (10) according to any one of the preceding claims 4 to 8, **characterized in that** the recess (34, 35, 51) of the finger flange (20) has an inner contour which at least in part has the exactly complementary shape to the outer contour of the at least one projection (11).

10. The syringe (10) according to any one of the preceding claims 4 to 9, **characterized in that** the recess of the finger flange (20) at least in part has an oversize compared to the projection (11).

11. The syringe (10) according to any one of the preceding claims 4 to 10, **characterized in that** the recess of the finger flange (20) in the contact area to the syringe body (12) is at least partially made of an elastic material in order to compensate for geometric deviations of the syringe body (12).

12. The syringe (10) according to any one of the preceding claims 4 to 11, **characterized in that** at least one finger flange member encloses the circumferential surface of the syringe body over an area corresponding to an opening angle α of at most 180°, preferably less than 180° and preferably less than 175°.

13. The syringe (10) according to any one of the preceding claims 4 to 12, **characterized in that**, in their closed position, the finger flange members are latched and/or non-releasably locked to one another.

14. The syringe (10) according to any one of the preceding claims 4 to 13, **characterized in that** the finger flange (20) comprises an elastic material, comprising PP, PE, POM, PBT, PA, PES, TPE, or glass fibre reinforced plastics.

## Revendications

1. Bride pour les doigts (20), en particulier pour la liaison par force et/ou par forme avec l'extrémité proximale d'un corps de seringue (12), dotée d'un évidement ou d'une ouverture de passage pour le passage d'une tige de piston, qui est réalisée d'une seule pièce, comprenant au moins un premier et un deuxième élément de bride pour les doigts (22, 23, 41, 42) qui sont reliés l'un à l'autre de manière mobile, de préférence de manière mobile en rotation, un verrouillage inséparable des éléments de bride pour les doigts étant prévu, et la bride pour les doigts (20) étant réalisée avec un élément empêchant le retrait de la tige de piston (18).

2. Bride pour les doigts (20) selon la revendication précédente, **caractérisée en ce que** les au moins premier et deuxième éléments de bride pour les doigts (22, 23, 41, 42) sont reliés l'un à l'autre au moyen d'une articulation, de préférence par matière au moyen d'une feuille ou d'un film d'articulation.

3. Bride pour les doigts (20) selon l'une des deux revendications précédentes, **caractérisée en ce que** la bride pour les doigts peut empêcher le retrait et/ou la rotation de la tige de piston et/ou du bouchon.

4. Seringue (10), comprenant un corps de seringue (12) cylindrique avec une extrémité distale et une extrémité proximale, ainsi qu'une bride pour les doigts (20) dotée d'au moins une surface d'appui pour un doigt selon l'une des revendications précédentes, dans laquelle
- le corps de seringue (12) comprend une chambre cylindrique destinée à accueillir une substance liquide,
- l'extrémité proximale du corps de seringue (12) présente une ouverture (19) à travers laquelle on peut faire passer une tige de piston (18),
- l'extrémité proximale du corps de seringue comprend par ailleurs au moins une saillie (11) qui avance radialement vers l'extérieur et dépasse par rapport à la surface enveloppe du corps de seringue,, sachant que
- la bride pour les doigts (20) est réalisée d'une seule pièce,
- la bride pour les doigts (20) comprend au moins un premier et un deuxième élément de bride pour les doigts (22, 23, 41, 42) qui sont reliés de manière mobile l'un à l'autre et peuvent être déplacés d'une position ouverte à une position fermée,
- en position fermée, la bride pour les doigts (20) est liée par force au moins en partie à la saillie (11), au nombre d'au moins une, et entoure la saillie (11) au moins en partie de manière à ce que dans la position fermée, un déplacement axial de la bride pour les doigts (20) par rapport au corps de seringue (12) soit empêché, sachant qu'un verrouillage inséparable des éléments de bride pour les doigt est prévu, sachant que les éléments de bride pour les doigts ne peuvent pas être séparés les uns des autres sans être détruits, sachant que la bride pour les doigts (20) est réalisée avec un élément empêchant le retrait de la tige de piston (18), et sachant que
- la bride pour les doigts (20) présente un évidement pour le passage de la tige de piston (18).

5. Seringue (10) selon la revendication précédente, **caractérisée en ce que** dans la position fermée, la bride pour les doigts (20) est reliée au moins en partie par forme à la saillie (11), au nombre d'au moins une.

6. Seringue (10) selon l'une des revendications précédentes 4 ou 5, **caractérisée en ce qu'**une tige de piston (18) destinée à être reliée à un bouchon (17) dans la chambre, ou une tige de piston dotée d'un bouchon peut être introduite à travers les ouvertures (19) à l'extrémité proximale du corps de seringue (12) et de la bride pour les doigts (20).

7. Seringue (10) selon l'une des revendications précédentes 4 à 6, **caractérisée en ce que** le premier et le deuxième élément de bride pour les doigts (22, 23, 41, 42) sont reliés l'un à l'autre avec possibilité de rotation au moyen d'une articulation (33, 43).

8. Seringue (10) selon l'une des revendications précédentes 4 à 7, **caractérisée en ce qu'**au moins le premier et le deuxième élément de bride pour les doigts (22, 23, 41, 42) sont reliés l'un à l'autre par matière au moyen d'une feuille ou d'un film d'articulation.

9. Seringue (10) selon l'une des revendications précédentes 4 à 8, **caractérisée en ce que** l'évidement (34, 35, 51) de la bride pour les doigts (20) présente un contour interne qui est réalisé au moins en partie avec ajustement serré complémentaire du contour externe de la saillie (11), au nombre d'au moins une.

10. Seringue (10) selon l'une des revendications précédentes 4 à 9, **caractérisée en ce que** l'évidement de la bride pour les doigts (20) présente au moins en partie un surdimensionnement par rapport à la saillie (11).

11. Seringue (10) selon l'une des revendications précédentes 4 à 10, **caractérisée en ce que** l'évidement de la bride pour les doigts (20) est formé, dans la zone de contact avec le corps de seringue (12), au moins en partie par un matériau élastique, aux fins de compenser des écarts géométriques du corps de seringue (12).

12. Seringue (10) selon l'une des revendications précédentes 4 à 11, **caractérisée en ce qu'**au moins un élément de bride pour les doigts entoure la surface périphérique du corps de seringue (12), dans une région qui correspond à un angle d'ouverture **α** d'au maximum 180°, de préférence inférieur à 180° et de préférence inférieur à 175°.

13. Seringue (10) selon l'une des revendications précédentes 4 à 12, **caractérisée en ce que** dans la position fermée, les éléments de bride pour les doigts sont encliquetés et/ou verrouillés de manière inséparable l'un avec l'autre.

14. Seringue (10) selon l'une des revendications précédentes 4 à 13, **caractérisée en ce que** la bride pour les doigts (20) comprend un matériau élastique, englobant PP, PE, POM, PBT, PA, PES, TPE ou des matières plastiques renforcées par fibres de verre.
